# EUROPEAN PATENT APPLICATION

(11) **EP 4 826 658 A1**
(43) Date of publication of application: **21.10.2026**
(21) Application number: 26173156.6
(22) Date of filing: 17.04.2026
(51) Int. Cl.: G06T 12/00

(54) **MEDICAL IMAGING METHOD, NON-TRANSITORY STORAGE MEDIUM, AND COMPUTER DEVICE**

(30) Priority: 17.04.2025 CN 202510486926
(71) Applicant: Shanghai United Imaging Healthcare Co., Ltd., Shanghai 201807 (CN)
(72) Inventor: REN, Jin, Shanghai, 201807 (CN); LIU, Yilin, Shanghai, 201807 (CN); HE, Liuchun, Shanghai, 201807 (CN); LV, Yang, Shanghai, 201807 (CN); DONG, Yun, Shanghai, 201807 (CN)
(74) Representative: Murgitroyd & Company

(57) **Abstract**

The present application discloses a medical imaging method, a non-transitory storage medium, and a computer device. The medical imaging method includes obtaining first double-coincidence data and second double-coincidence data of a scanned subject, wherein the first double-coincidence data is determined based on double-coincidence events generated by nuclides in a first tracer and double-coincidence events generated by nuclides in a second tracer in the subject, and the second double-coincidence data is determined based on double-coincidence events from triple-coincidence events generated by the nuclides in the second tracer. The medical imaging method further includes obtaining, by iterations, a target activity distribution of the first tracer and a target activity distribution of the second tracer, based on the first double-coincidence data and the second double-coincidence data.

## Description

### TECHNICAL FIELD

The present application relates to the technical field of medical imaging, and in particular to a medical imaging method, a non-transitory storage medium, and a computer device.

### BACKGROUND

With the development of medical technology, Positron Emission Tomography (PET) imaging, as an advanced nuclear medicine examination technique, uses radioactive tracers to label biological molecules, detects metabolic activities in the body of a subject, and generates detailed three-dimensional images, thereby providing an important basis for disease diagnosis.

In the related art, a single PET scan can only perform functional imaging with a single tracer, which has limited capability for characterizing complex biological activities. Although multiple PET scans can more comprehensively provide biological activity information, they entail high economic and time costs, and registration problems between multiple scans also affect the final imaging quality. It can be seen that the related art has the problem that imaging quality and imaging efficiency of nuclear medicine images cannot be reconciled.

### SUMMARY

In a first aspect, a medical imaging method is provided in the present application. The medical imaging method includes obtaining first double-coincidence data of a scanned subject and second double-coincidence data of the subject, where the first double-coincidence data is determined based on double-coincidence events generated by nuclides in a first tracer and double-coincidence events generated by nuclides in a second tracer in the subject, and the second double-coincidence data is determined based on double-coincidence events from triple-coincidence events generated by the nuclides in the second tracer;
obtaining, by iterations, a target activity distribution of the first tracer and a target activity distribution of the second tracer, based on the first double-coincidence data and the second double-coincidence data, by performing the following sub-steps:
obtaining, by iterations, activity distributions of the first tracer and activity distributions of the second tracer, based on the first double-coincidence data and the second double-coincidence data;
obtaining, for each of the iterations, a respective evaluation parameter reflecting an accuracy of the iteration, based on the activity distribution of the first tracer and the activity distribution of the second tracer both obtained in the iteration; and
determining a target activity distribution of the first tracer and a target activity distribution of the second tracer, based on the evaluation parameters respectively corresponding to the iterations, when a stopping criterion for iteration is satisfied.

In a second aspect, a computer device is further provided in the present application. The computer device includes a memory and a processor, where the memory stores a computer program, and when the processor executes the computer program, the processor implements the following steps:
obtaining first double-coincidence data of a subject and second double-coincidence data of the subject, wherein the first double-coincidence data is determined based on double-coincidence events generated by nuclides in a first tracer and double-coincidence events generated by nuclides in a second tracer in the subject, and the second double-coincidence data is determined based on double-coincidence events from triple-coincidence events generated by the nuclides in the second tracer;
obtaining, by iterations, a target activity distribution of the first tracer and a target activity distribution of the second tracer, based on the first double-coincidence data and the second double-coincidence data, by performing the following sub-steps:
   obtaining, by iterations, activity distributions of the first tracer and activity distributions of the second tracer, based on the first double-coincidence data and the second double-coincidence data;
   obtaining, for each of the iterations, a respective evaluation parameter reflecting an accuracy of the iteration, based on the activity distribution of the first tracer and the activity distribution of the second tracer both obtained in the iteration; and
   determining a target activity distribution of the first tracer and a target activity distribution of the second tracer, based on the evaluation parameters respectively corresponding to the iterations, when a stopping criterion for iteration is satisfied.

In a third aspect, a non-transitory computer-readable storage medium is further provided in the present application. The non-transitory computer-readable storage medium has a computer program stored thereon, where the computer program, when executed by a processor, causes the processor to implement the following steps:
obtaining first double-coincidence data of a subject and second double-coincidence data of the subject, wherein the first double-coincidence data is determined based on double-coincidence events generated by nuclides in a first tracer and double-coincidence events generated by nuclides in a second tracer in the subject, and the second double-coincidence data is determined based on double-coincidence events from triple-coincidence events generated by the nuclides in the second tracer;
obtaining, by iterations, a target activity distribution of the first tracer and a target activity distribution of the second tracer, based on the first double-coincidence data and the second double-coincidence data, by performing the following sub-steps:
   obtaining, by iterations, activity distributions of the first tracer and activity distributions of the second tracer, based on the first double-coincidence data and the second double-coincidence data;
   obtaining, for each of the iterations, a respective evaluation parameter reflecting an accuracy of the iteration, based on the activity distribution of the first tracer and the activity distribution of the second tracer both obtained in the iteration; and
   determining a target activity distribution of the first tracer and a target activity distribution of the second tracer, based on the evaluation parameters respectively corresponding to the iterations, when a stopping criterion for iteration is satisfied.

### BRIEF DESCRIPTION OF DRAWINGS

FIG. 1 is a schematic flowchart of a medical imaging method in an embodiment.
FIG. 2 is a schematic flowchart of steps for obtaining an iteration result in an embodiment.
FIG. 3 is a schematic flowchart of another medical imaging method in an embodiment.
FIG. 4 is a structural block diagram of a medical imaging apparatus in an embodiment.
FIG. 5 is a diagram of an internal structure of a computer device in an embodiment.
FIG. 6 is a diagram of an internal structure of another computer device in an embodiment.

### DETAILED DESCRIPTION

In order to make the objectives, technical solutions, and advantages of the present application clearer, the present application will be further described in detail below with reference to the accompanying drawings and embodiments. It should be understood that the specific embodiments described herein are only used to explain the present application and are not used to limit the present application.

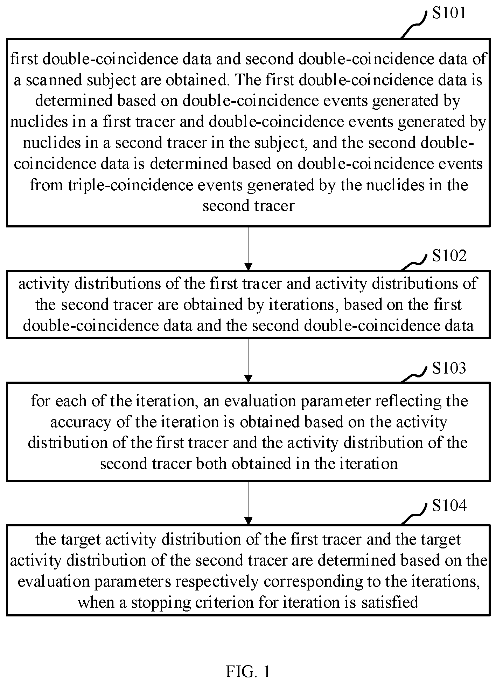

In an embodiment, as shown in FIG. 1, a medical imaging method is provided. This embodiment is described by taking the application of the medical imaging method to a terminal as an example. It can be understood that the medical imaging method can also be applied to a server, and can further be applied to a system including a terminal and a server, and implemented through the interaction between the terminal and the server. In this embodiment, the medical imaging method includes the following Step S101 to Step S104.

At Step S101, first double-coincidence data and second double-coincidence data of a scanned subject are obtained. The first double-coincidence data is determined based on double-coincidence events generated by nuclides in a first tracer and double-coincidence events generated by nuclides in a second tracer in the subject, and the second double-coincidence data is determined based on double-coincidence events from triple-coincidence events generated by the nuclides in the second tracer.

The subject may be a living organism having vital functions, such as a human body or an animal. The double-coincidence data, such as the first double-coincidence data or the second double-coincidence data, may include at least one piece of triplet data in the form of (*p*₁, *p*₂, *t*₁₂). A piece of triplet data (*p*₁, *p*₂, *t*₁₂) can characterize one double-coincidence event. A piece of triple-coincidence data may be a combination of three pieces of double-coincidence data in the form of {(*p*₁, *p*₂, *t*₁₂), (*p*₁*, p*₃*, t*₁₃), (*p*₂, *p*₃, *t*₂₃)}. A triple-coincidence event may be characterized by a combination of three pieces of double-coincidence data. *p*₁ and *p*₂ can characterize the spatial positions (detected photon positions), respectively, at which photons *p*₁ and *p*₂ are detected by detectors. *t*₁₂ can characterize the Time of Flight (ToF) between the detected times of the photons *p*₁ and *p*₂. Given that the propagation speed of photons is extremely fast, the ToF between emission and reception of a single photon is almost zero, and therefore, the ToF between multiple photons can be understood as a difference between the moments at which the multiple photons are detected by the detectors. A tracer, such as the first tracer or the second tracer, can be a substance that is added to a certain system (for example, a biological reaction system or a chemical reaction system) to identify changes in the subject under study. Through detectable nuclides, concentration, or other signals, the tracer can indicate or inversely infer the reactions or changes of the system.

As described above, in the related art, a single PET-based scan can only perform functional imaging on a single tracer, which has limited capability for characterizing complex biological activities, while multiple PET-based scans, though able to more comprehensively provide biological activity information, entail high economic and time costs, and registration problems between multiple scans also affect the final imaging quality. In view of this, the inventors have found in practice that single-scan simultaneous imaging based on PET with multiple tracers has significant clinical value.

In an embodiment, two different tracers can be injected into the subject. For ease of distinction, the two tracers are referred to as the first tracer and the second tracer, respectively.

In an embodiment, the first tracer and the second tracer can be injected into the subject simultaneously or injected into the subject successively. In the case of successive injection, the injection can be performed based on a predetermined injection interval (i.e., the time interval between the injection of the first tracer and the injection of the second tracer) and/or an injection sequence. For example, the injection interval and the injection sequence can be determined according to clinical guidelines. The injection interval is less than a preset threshold. The preset threshold can be determined according to an accuracy of the target activity distribution of the first tracer and the accuracy of the target activity distribution of the second tracer obtained under different injection intervals. The accuracy can be evaluated by comparing the target activity distribution of the first tracer and the target activity distribution of the second tracer obtained under different injection intervals against respective reference activity distributions (for example, a ground-truth distribution in a simulation experiment or a reference distribution obtained by a single scan of a single tracer). For example, the accuracy may be characterized by an image similarity metric such as a structural similarity index (SSIM), a peak signal-to-noise ratio (PSNR), or a mean squared error (MSE). In an implementation, a maximum injection interval that causes the accuracy to satisfy a preset requirement may be determined experimentally, and the maximum injection interval may be taken as the preset threshold.

In an embodiment, since the nuclides in some tracers probabilistically emit prompt-y rays while undergoing *β*⁺ decay, triple-coincidence events are generated after positron annihilation, which can separate dual-nuclide signals during the process of imaging of a PET, simultaneously combined with dual tracers. For this purpose, the first tracer used in the present application can only generate pure double-coincidence events, such as a *β*⁺ tracer. The second tracer can simultaneously generate double-coincidence events and triple-coincidence events, such as a *β*⁺*γ* tracer.

In an embodiment, by performing data obtainment and data extraction on the double-coincidence events generated by the first tracer and the second tracer, the first double-coincidence data can be obtained. By performing data obtainment and data extraction on the triple-coincidence events generated by the second tracer, the double-coincidence events contained in the triple-coincidence events can be determined, and thereby the corresponding second double-coincidence data can be obtained.

Specifically, the first double-coincidence data may be understood as double-coincidence event data remaining after screening out the double-coincidence events contained in the triple-coincidence events from event data of the double-coincidence events obtained by scanning the subject. That is, the first double-coincidence data includes event data corresponding to double-coincidence events (pure double-coincidence events) generated by the nuclides of the first tracer, and event data corresponding to double-coincidence events (other than the double-coincidence events contained in the triple-coincidence events) generated by the nuclides of the second tracer. The second double-coincidence data can be understood as follows: for each triple-coincidence event generated by the nuclides of the second tracer, the triple-coincidence event corresponds to three double-coincidence events, and after the two double-coincidence events containing a prompt gamma photon (prompt-γ) are removed from the three double-coincidence events, the event data corresponding to the remaining one double-coincidence event constitutes a portion of the second double-coincidence data; the above removal operation is repeated for the triple-coincidence events, and the event data corresponding to all the obtained remaining double-coincidence events constitutes the second double-coincidence data.

In an embodiment, both the first double-coincidence data and the second double-coincidence data can be derived from a pair of *γ* photons generated after electron-pair annihilation. Two opposing detectors in a medical scanning system can detect paired *γ* photons, and the output electrical pulses, after being screened by energy discrimination (for example, screened through an energy discrimination window), can be sent into a coincidence circuit when the output electrical pulses meet the energy requirement. A referencing line connecting the two detectors can be a Line of Response. The Line of Response contains its own positional information. Accordingly, the first double-coincidence data can characterize the double-coincidence events detected by the detectors after decay of the nuclides in the first tracer in the subject. The second double-coincidence data can characterize the double-coincidence events detected by the detectors after decay of the nuclides in the second tracer in the subject.

In some embodiments, under a single scan, the detectors can simultaneously obtain the first double-coincidence data and the second double-coincidence data of the subject. In other words, both the first double-coincidence data and the second double-coincidence data can be obtained simultaneously based on the scan data obtained by a single scan. Obtaining the two sets of double-coincidence data in this way can improve the degree of matching between the first doublecoincidence data and the second double-coincidence data, and improve the accuracy of the activity distributions of the two tracers, which are subsequently obtained by iterations, based on the first double-coincidence data and the second double-coincidence data.

Steps S102 to S104 collectively obtain, by iterations, a target activity distribution of the first tracer and a target activity distribution of the second tracer, based on the first double-coincidence data and the second double-coincidence data.

At Step S102, activity distributions of the first tracer and activity distributions of the second tracer are obtained by iterations, based on the first double-coincidence data and the second double-coincidence data.

In an embodiment, the activity distribution of the first tracer in the subject and the activity distribution of the second tracer in the subject can be determined based on the first double-coincidence data and the second double-coincidence data. Specifically, the activity distribution of the first tracer may be a *β*⁺ tracer that only produces double-coincidence events, and the activity distribution of the second tracer may be a *β*⁺γ tracer that simultaneously produces double-coincidence events and triple-coincidence events. The activity distribution of a tracer can be presented in the form of an activity distribution image of the tracer, and is a core output of nuclear medicine imaging (such as PET or SPECT), which can directly reflect a three-dimensional spatial distribution and a concentration level of the radioactive tracer in the subject and can be used to reveal a physiological function and a metabolic status of a specific organ, tissue, or lesion. In an implementation, the first tracer and the second tracer both injected into the same subject can have respective activity distributions that are mutually coupled, that is, the activity distribution of one tracer can affect the activity distribution of the other tracer. In view of this, in this embodiment, the activity distribution of the first tracer and the activity distribution of the second tracer can be jointly obtained by iterations, based on the first double-coincidence data and the second double-coincidence data. In other words, in each iteration, a possible mutual influence between the first tracer and the second tracer, as well as a data relationship between the activity distributions of the two tracers, can be jointly obtained, and then the activity distribution of the first tracer and the activity distribution of the second tracer can be obtained in each iteration, based on the data relationship. The activity distribution can be characterized by a function *F*(*u*)*,* where *u* can represent a coordinate point *p*(*x, y, z*) in space, and *F*(*p*(*x, y, z*)) can represent the activity of the tracer at the coordinate point *p*(*x, y, z*).

In some embodiments, the first double-coincidence data and the second double-coincidence data can be input into a pre-constructed dual-tracer simultaneous imaging model, and the activity distribution of the first tracer and the activity distribution of the second tracer are iteratively reconstructed simultaneously by the dual-tracer simultaneous imaging model. In some implementations, a convergent fixed-point iteration algorithm can be used to iterate the activity distribution of the first tracer and the activity distribution of the second tracer, such as a Fixed-Point Proximity Algorithm related to a proximal operator, a splitting (Douglas-Rachford) method, a Primal-Dual method, an inexact Uzawa method, and the like. Other iteration algorithms may also be used, such as an Alternating Direction Method of Multipliers (ADMM) or an Ordered Subsets Expectation Maximization (OSEM) method.

At Step S103, for each of the iteration, an evaluation parameter reflecting the accuracy of the iteration is obtained based on the activity distribution of the first tracer and the activity distribution of the second tracer both obtained in the iteration.

In an implementation, for the activity distribution of the first tracer and the activity distribution of the second tracer both obtained in each iteration, the accuracy of the iteration result can be detected to obtain the evaluation parameter reflecting the accuracy of the iteration.

In some embodiments, based on the activity distribution of the first tracer and the activity distribution of the second tracer obtained by the iteration, a forward problem of a physical model can be solved, so as to obtain simulated measurement data through calculation. Then, based on the difference between the simulated measurement data and the actual measurement data, the evaluation parameter reflecting the accuracy of the iteration can be obtained. In addition to comparing the difference between the simulated measurement data and the actual measurement data, statistical tests such as a t-test or an F-test can also be performed to determine whether there is any statistically significant difference between the simulated measurement data and the actual measurement data. When there is no significant difference between the simulated measurement data and the actual measurement data, it indicates that the iteration result is correct and reasonable under the framework of the physical model and has relatively high accuracy. Solving the forward problem of the physical model can be understood as a process of predicting, in the case where physical characteristics of a system and relevant parameters are known, the output or behavior of the system by physical laws and/or mathematical methods that conform to natural laws. In this embodiment, in an aspect, one or more pieces of measurable physical information associated with the activity distribution of the first tracer and the activity distribution of the second tracer can be determined, and the actual measurement data of the measurable physical information can be obtained; in another aspect, the simulated measurement data of measurable physical information can be calculated based on the first tracer, the second tracer, and known parameters associated therewith, and then the evaluation parameter corresponding to the iteration can be determined based on the simulated measurement data and the actual measurement data.

In an embodiment, since the activity distribution of the first tracer and the activity distribution of the second tracer can be presented in the form of images, evaluation based on image features can be used to obtain the evaluation parameter of each iteration. The evaluation parameter includes at least one of a Peak Signal-to-Noise Ratio (PSNR), a Mean Squared Error (MSE), a Learned Perceptual Image Patch Similarity (LPIPS), and a Structural Similarity Index Measure. Taking the Structural Similarity Index Measure (SSIM) as an example of the evaluation metric, the similarity of image features can be comprehensively evaluated from three aspects of image luminance, contrast, and structure. For an activity distribution image that can represent a tracer activity distribution, statistics such as mean, variance, and covariance of designated regions to be compared in the activity distribution image can be determined, and then the SSIM can be determined based on the statistics of different designated regions. The value of SSIM is in the range of [-1,1]. The closer the value of SSIM is to 1, the more similar in structure the activity distribution image obtained by iteration is to the real image, that is, the higher the accuracy of imaging. In another example, edge detection and edge comparison can be performed, that is, the edge information of the iteration result image of the iteration result and the edge information of the reference image are extracted by an edge detection algorithm, and then features, such as the position, intensity, and continuity, of the edges in an iteration result image and a reference image are compared, respectively. When the features match, it can be determined that the iteration result has relatively high accuracy.

At Step S104, the target activity distribution of the first tracer and the target activity distribution of the second tracer are determined based on the evaluation parameters respectively corresponding to the iterations, when a stopping criterion for iteration is satisfied.

In an embodiment, through the relevant iteration algorithm, the activity distribution of the first tracer and the activity distribution of the second tracer can be simultaneously iterated multiple times, and the evaluation parameter corresponding to each iteration can be obtained. When the stopping criterion for iteration is satisfied (for example, when the iteration result converges or the number of iterations reaches a preset number, the stopping criterion for iteration is determined to be satisfied), the activity distribution of the first tracer and the activity distribution of the second tracer whose accuracy of iteration meets an accuracy requirement can be determined from the iterations, based on the evaluation parameters respectively corresponding to the iterations. In some examples, an target activity distribution of the first tracer and an target activity distribution of the second tracer with the highest accuracy of iteration can be determined based on the evaluation parameters, and the target activity distribution of the first tracer and the target activity distribution of the second tracer can be taken as the target activity distribution of the first tracer and the target activity distribution of the second tracer.

In the above medical imaging method, the data to be processed can be obtained by obtaining the first double-coincidence data of the subject and the second double-coincidence data of the subject. The activity distribution of the first tracer and the activity distribution of the second tracer can be obtained by performing iterations, based on the first double-coincidence data and the second double-coincidence data. The evaluation parameter of each iteration, reflecting the accuracy of the iteration, can be obtained based on the activity distribution of the first tracer and the activity distribution of the second tracer both obtained by the iteration. When the stopping criterion for iteration is satisfied, the target activity distribution of the first tracer and the target activity distribution of the second tracer are determined based on the evaluation parameters respectively corresponding to the iterations, so as to obtain an optimal target activity distribution of the first tracer and an optimal target activity distribution of the second tracer meeting the accuracy requirement.

In this embodiment, by injecting dual tracers into the subject and simultaneously performing modelling, the activity distributions of the dual tracers in the subject are determined, so that more biological activity information of the subject can be provided while reducing the number of scans, effectively reconciling the imaging quality and the imaging efficiency of nuclear medicine images.

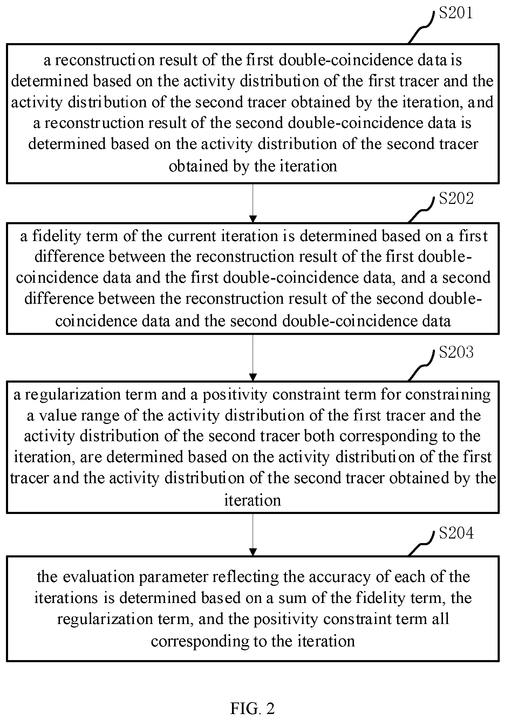

In an embodiment, as shown in FIG. 2, at Step S103, for each of the iteration, an evaluation parameter reflecting the accuracy of the iteration is obtained based on the activity distribution of the first tracer and the activity distribution of the second tracer both obtained in the iteration, can include the following Step S201 to Step S204.

At Step S201, a reconstruction result of the first double-coincidence data is determined based on the activity distribution of the first tracer and the activity distribution of the second tracer obtained by the iteration, and a reconstruction result of the second double-coincidence data is determined based on the activity distribution of the second tracer obtained by the iteration.

The first double-coincidence data can include double-coincidence event data corresponding to the double-coincidence events generated by the nuclides in the first tracer, and double-coincidence data corresponding to the double-coincidence events from the triple-coincidence events generated by the nuclides in the second tracer. Therefore, both the activity distribution of the first tracer and the activity distribution of the second tracer can affect the actual observed value of the first double-coincidence data. The first double-coincidence data can be a result formed under the joint action of the activity distribution of the first tracer and the activity distribution of the second tracer. Based on this, the first double-coincidence data can be reconstructed based on the activity distribution of the first tracer and the activity distribution of the second tracer obtained by the iteration, to determine the reconstruction result of the first double-coincidence data.

In addition, the activity distribution of the second tracer affects not only the first double-coincidence data but also the second double-coincidence data. Since the second double-coincidence data is determined based on the double-coincidence events from the triple-coincidence events generated by the nuclides in the second tracer, for the second double-coincidence data, it can be assumed that the activity distribution of the first tracer does not influence the actual observed value of the second double-coincidence data. Based on this, the reconstruction result of the second double-coincidence data can be determined based on the activity distribution of the second tracer obtained by the iteration.

At Step S202, a fidelity term of the current iteration is determined based on a first difference between the reconstruction result of the first double-coincidence data and the first double-coincidence data, and a second difference between the reconstruction result of the second double-coincidence data and the second double-coincidence data.

In an embodiment, after the reconstruction result of the first double-coincidence data and the reconstruction result of the second double-coincidence data are obtained, the deviation between reconstructed values and observed values can be determined. Specifically, the first difference between the reconstruction result of the first double-coincidence data and the first double-coincidence data, and the second difference between the reconstruction result of the second double-coincidence data and the second double-coincidence data, can be determined. Then, based on the first difference and the second difference, the fidelity term reflecting the degree of matching between the double-coincidence data reconstruction results and the actual double-coincidence data may be determined. The first difference and the second difference are between the reconstructed values and the observed values.

In an embodiment, the fidelity term is used to measure the degree of deviation between the reconstructed values and the observed values of each of the iterations. Specifically, the reconstruction result of the first double-coincidence data can first be determined based on the activity distribution of the first tracer and the activity distribution of the second tracer obtained by each of the iterations; and the reconstruction result of the second double-coincidence data can be determined based on the activity distribution of the second tracer obtained by each of the iterations. The reconstruction result of the first double-coincidence data can be understood as a set of expected count values on Lines of Response corresponding to the first double-coincidence data, obtained by simulating a forward projection operation of a physical model based on the activity distribution of the first tracer and the activity distribution of the second tracer obtained by each of the iterations; and the reconstruction result of second double-coincidence data can be understood as a set of expected count values on Lines of Response corresponding to the second double-coincidence data, obtained by simulating a forward projection operation of the physical model based on the activity distribution of the second tracer obtained by each of the iterations.

At Step S203, a regularization term and a positivity constraint term for constraining a value range of the activity distribution of the first tracer and the activity distribution of the second tracer both corresponding to the iteration, are determined based on the activity distribution of the first tracer and the activity distribution of the second tracer obtained by the iteration.

In an implementation, since the activity distribution of the first tracer and the activity distribution of the second tracer are positive numbers, the positivity constraint term for constraining the value ranges of the activity distribution of the first tracer and the activity distribution of the second tracer can be obtained in this step. In an example, for the positivity constraint term 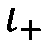 (*f*₂, *f*₃) of the activity distribution of the first tracer and the activity distribution of the second tracer, the function 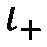 can be defined by the following Formula (1): ${ι}_{+} \left({f}_{2}, {f}_{3}\right) : = \left\{\begin{matrix}0 , & {f}_{2} ≽ 0 and {f}_{3} ≽ 0 \\ + ∞ , & otherwise\end{matrix}\right.$ (1) where *f*₂ is the activity distribution of the first tracer , and *f*₃ is the activity distribution of the second tracer.

In another aspect, since there is noise in the double-coincidence data, in this embodiment, the corresponding regularization term can also be determined based on the activity distribution of the first tracer and the activity distribution of the second tracer both obtained by the iteration, and the noise can be suppressed by adding the regularization term to improve the iteration effect.

In some examples, the regularization term Reg(*f*₂, *f*₃) corresponding to the activity distribution of the first tracer and the activity distribution of the second tracer may be selected from a group of *Lₚ*-TV total variation, structure tensor total variation (STV), and Relative Difference Penalty.

At Step S204, the evaluation parameter reflecting the accuracy of each of the iterations is determined based on a sum of the fidelity term, the regularization term, and the positivity constraint term all corresponding to the iteration.

Then, for the activity distribution of the first tracer and the activity distribution of the second tracer obtained by each of the iterations, the evaluation parameter reflecting the accuracy of the iteration can be determined based on the sum of the fidelity term, the regularization term, and the positivity constraint term, which are all corresponding to the iteration.

With respect to the evaluation parameter obtained in the above, the value of the evaluation parameter can be inversely proportional to the accuracy of iteration, that is, the smaller the value of the evaluation parameter, the higher the accuracy of iteration. Subsequently, by comparing the evaluation parameters of the iterations, the iteration result (i.e., the activity distribution of the first tracer and the activity distribution of the second tracer) at which the evaluation parameter reaches the minimum can be determined, and the target activity distribution of the first tracer and the target activity distribution of the second tracer of which the respective evaluation parameters reach the minimums can be taken as the target activity distribution of the first tracer and the target activity distribution of the second tracer.

In this embodiment, by determining the evaluation parameter reflecting the accuracy of each of the iterations, based on the sum of the fidelity term, the regularization term, and the positivity constraint term all corresponding to the iteration, the simultaneous imaging of dual tracers can be physically modelled and a corresponding mathematical model can be constructed, and the accuracy of iteration of the tracer activity distributions in each of the iterations can be detected, so that the simultaneous imaging of the dual tracers can be performed relatively accurately.

In an embodiment, at Step S201, determining the reconstruction result of the first double-coincidence data based on the activity distribution of the first tracer and the activity distribution of the second tracer both obtained by the iteration, and determining the reconstruction result of the second double-coincidence data based on the activity distribution of the second tracer obtained by the iteration, can include the following steps:
determining the reconstruction result of the first double-coincidence data based on the activity distribution of the first tracer obtained by the iteration, a first preset influence factor of the activity distribution of the first tracer on the first double-coincidence data, the activity distribution of the second tracer obtained by the iteration, and a second preset influence factor of the activity distribution of the second tracer on the first double-coincidence data; and determining the reconstruction result of the second double-coincidence data based on the activity distribution of the second tracer obtained by the iteration and a third preset influence factor of the activity distribution of the second tracer on the second double-coincidence data.

Specifically, the first double-coincidence data can include double-coincidence event data corresponding to the double-coincidence events generated by the nuclides in the first tracer and double-coincidence data corresponding to the double-coincidence events from the triple-coincidence events generated by the nuclides in the second tracer. Both the activity distribution of the first tracer and the activity distribution of the second tracer can thus affect the actual observed values of the first double-coincidence data, and the first double-coincidence data can be a result formed under the joint action of the activity distribution of the first tracer and the activity distribution of the second tracer.

Based on this, the preset influence factor of the activity distribution of the first tracer on the first double-coincidence data and the preset influence factor of the activity distribution of the second tracer on the first double-coincidence data can be predetermined. For ease of distinction, one of the preset influence factors is referred to as the first preset influence factor, and the other is referred to as the second preset influence factor. The first preset influence factor can characterize the contribution or a degree of contribution of the activity distribution of the first tracer to forming the first double-coincidence data. In some examples, the first preset influence factor may be a forward projection operator *A*₂₂ that maps the activity distribution of the first tracer to the double-coincidence event data generated by the nuclides of the first tracer. Similarly, the second preset influence factor can characterize the contribution or a degree of contribution of the activity distribution of the second tracer to forming the first double-coincidence data. In some examples, the second preset influence factor may be a forward projection operator *A*₃₂ that maps the activity distribution of the second tracer to the double-coincidence event data generated by the nuclides of the second tracer (i.e., the double-coincidence event data corresponding to the double-coincidence events other than the triple-coincidence events).

In addition, the activity distribution of the second tracer affects not only the actual observed values of the first double-coincidence data, but also the actual observed values of the second double-coincidence data. Since the second double-coincidence data is determined based on the double-coincidence events from the triple-coincidence events generated by the nuclides in the second tracer, for the second double-coincidence data, it may be assumed that the activity distribution of the first tracer does not influence the actual observed values of the second double-coincidence data.

Based on this, the preset influence factor of the activity distribution of the second tracer on the second double-coincidence data can also be predetermined. For ease of distinction, this preset influence factor is referred to as the third preset influence factor. The third preset influence factor can characterize the contribution or a degree of contribution of the activity distribution of the second tracer to forming the second double-coincidence data. In some examples, the third preset influence factor may be a forward projection operator *A*₃ that maps the activity distribution of the second tracer to the second double-coincidence data of the second tracer.

Accordingly, based on the activity distribution of the first tracer obtained by the iteration, the first preset influence factor, the activity distribution of the second tracer obtained by the iteration, and the second preset influence factor, the first double-coincidence data can be reconstructed by calculation to obtain the reconstruction result of the first double-coincidence data. In addition, based on the activity distribution of the second tracer obtained by the iteration and the third preset influence factor, the second double-coincidence data can be reconstructed by calculation to obtain the reconstruction result of the second double-coincidence data.

In this embodiment, a physical model can be constructed, according to relationships between the activity distribution of the first tracer and the first double-coincidence data, and between the activity distribution of the second tracer and the second double-coincidence data, in combination with the first preset influence factor, the second preset influence factor, and the third preset influence factor, which helps to obtain a relatively accurate reconstruction result of the first double-coincidence data and a relatively accurate reconstruction result of the second double-coincidence data, thereby detecting the accuracy of each of iterations.

In an embodiment, determining the reconstruction result of the first double-coincidence data based on the activity distribution of the first tracer obtained by the iteration, the first preset influence factor of the activity distribution of the first tracer on the first double-coincidence data, the activity distribution of the second tracer obtained by the iteration, and the second preset influence factor of the activity distribution of the second tracer on the first double-coincidence data, can include the following steps:
determining a double-coincidence data reconstruction result of the double-coincidence events generated by the nuclides in the first tracer based on a product of the activity distribution of the first tracer obtained by the iteration and the first preset influence factor of the activity distribution of the first tracer on the first double-coincidence data; determining a double-coincidence data reconstruction result of the double-coincidence events generated by the nuclides in the second tracer based on a product of the activity distribution of the second tracer obtained by the iteration and the second preset influence factor of the activity distribution of the second tracer on the first double-coincidence data; and determining the reconstruction result of the first double-coincidence data based on the double-coincidence data reconstruction result of the double-coincidence events generated by the nuclides in the first tracer and the double-coincidence data reconstruction result of the double-coincidence events generated by the nuclides in the second tracer.

In an embodiment, after the first preset influence factor and the second preset influence factor are obtained, in an aspect, the product of the activity distribution of the first tracer and the first preset influence factor can be determined, and this product can be taken as the double-coincidence data reconstruction result of the double-coincidence events generated by the nuclides in the first tracer; in another aspect, the product of the activity distribution of the second tracer and the second preset influence factor can be determined, and this product can be taken as the double-coincidence data reconstruction result of the double-coincidence events generated by the nuclides in the second tracer. Subsequently, the reconstruction result of the first double-coincidence data can be determined relatively quickly by combining the two double-coincidence data reconstruction results. This processing has relatively high interpretability, and the reconstruction result of the first double-coincidence data can be quickly determined merely by the product, and the processing is concise, effectively improving the iteration efficiency of the two tracer activity distributions.

In an embodiment, determining the reconstruction result of the first double-coincidence data based on the double-coincidence data reconstruction result of the double-coincidence events generated by the nuclides in the first tracer and the double-coincidence data reconstruction result of the double-coincidence events generated by the nuclides in the second tracer can include:
obtaining first scatter estimation information corresponding to the first double-coincidence data; and obtaining the reconstruction result of the first double-coincidence data based on a sum of the double-coincidence data reconstruction result of the double-coincidence events generated by the nuclides in the first tracer, the double-coincidence data reconstruction result of the double-coincidence events generated by the nuclides in the second tracer, and the first scatter estimation information.

In an implementation, a scatter estimation can be performed on the first double-coincidence data to obtain scatter estimation information. For ease of distinction, this scatter estimation information is referred to as the first scatter estimation information.

Specifically, when γ-rays pass through the subject, the direction of the γ-rays is deflected due to the interaction between the *γ* -rays and atoms, resulting in scattering. The scattering can cause the detected photon positions of some of the double-coincidence events to deviate from the actual decay positions, thereby constituting a bias in observed data. The first scatter estimation information can be a set of expected count values contributed by scattered events in the first double-coincidence data (for example, an expected value of a number of scattered events on a Line of Response), and is used to compensate for the bias in the reconstruction. Scatter estimation can be performed on the first double-coincidence data by using a Single Scatter Simulation (SSS), a Monte Carlo simulation, a tail-fitting-based method, or the like, to obtain the first scatter estimation information. In view of this, in this embodiment, the scatter estimation is performed on the first double-coincidence data, and the estimated first scatter estimation information, the double-coincidence data reconstruction result of the double-coincidence events generated by the nuclides in the first tracer, and the double-coincidence data reconstruction result of the double-coincidence events generated by the nuclides in the second tracer, are summed. The summation result is taken as the reconstruction result of the first double-coincidence data. As such, the first double-coincidence data can be reconstructed in consideration of the scattering, so that the reconstruction result is closer to the actual situation, and the iteration efficiency of the two tracer activity distributions is accelerated.

In an embodiment, determining the reconstruction result of the second double-coincidence data based on the activity distribution of the second tracer obtained by the iteration and the third preset influence factor of the activity distribution of the second tracer on the second double-coincidence data can include the following steps:
determining the double-coincidence data reconstruction result corresponding to the double-coincidence events from the triple-coincidence events of the second tracer based on a product of the activity distribution of the second tracer obtained by the iteration and the third preset influence factor of the activity distribution of the second tracer on the second double-coincidence data; and determining the reconstruction result of the second double-coincidence data based on the double-coincidence data reconstruction result corresponding to the double-coincidence events from the triple-coincidence events of the second tracer.

In practical applications, after the third preset influence factor is obtained, the product of the activity distribution of the second tracer and the third preset influence factor can be determined. This product can be taken as the double-coincidence data reconstruction result corresponding to the double-coincidence events from the triple-coincidence events of the second tracer. The reconstruction result of the second double-coincidence data can be determined based on this result. This processing has relatively high interpretability, the reconstruction result of the second double-coincidence data can be quickly determined merely by a product, and the processing is concise, thereby effectively improving the iteration efficiency of the two tracer activity distributions.

In an embodiment, determining the reconstruction result of the second double-coincidence data based on the double-coincidence data reconstruction result corresponding to the double-coincidence events from the triple-coincidence events of the second tracer can include the following steps:
obtaining second scatter estimation information corresponding to the second double-coincidence data; and obtaining the reconstruction result of the second double-coincidence data based on a sum of the double-coincidence data reconstruction result corresponding to the double-coincidence events from the triple-coincidence events of the second tracer and the second scatter estimation information.

In an embodiment, scatter estimation can be performed on the second double-coincidence data to obtain the scatter estimation information. For ease of distinction, this scatter estimation information is referred to as the second scatter estimation information. The second scatter estimation information can be a set of expected count values contributed by scattered events in the second double-coincidence data, and can be obtained by performing scatter estimation on the second double-coincidence data in accordance with the foregoing manner for obtaining the first scatter estimation information (for example, a Single Scatter Simulation, a Monte Carlo simulation, a tail-fitting-based method, or the like). In this embodiment, by performing scatter estimation on the second double-coincidence data, summing the estimated second scatter estimation information and the double-coincidence data reconstruction result corresponding to the double-coincidence events from the triple-coincidence events generated by the second tracer, and taking the summation result as the reconstruction result of the second double-coincidence data, the second double-coincidence data can be reconstructed in consideration of the scattering, so that the reconstruction result is closer to the actual situation, and the iteration efficiency of the dual-tracer activity distributions is accelerated.

In an embodiment, at Step S101, obtaining the first double-coincidence data of the subject and the second double-coincidence data of the subject can include the following Step S1011 to Step S1013.

At Step S1011, double-coincidence event data respectively corresponding to the double-coincidence events of the subject injected with the first tracer and the second tracer are obtained. The nuclides of the first tracer produce double-coincidence events. The nuclides of the second tracer produce double-coincidence events and triple-coincidence events. Each piece of the double-coincidence event data includes two detected photon positions and the ToF between the two detected photon positions.

In some embodiments, the first tracer can only generate double-coincidence events, such as a *β*⁺ tracer. The second tracer can simultaneously generate double-coincidence events and triple-coincidence events, such as a *β*⁺*γ* tracer.

In some embodiments, after the first tracer and the second tracer are injected into the subject, PET scanning can be performed on the subject, and by analyzing and extracting data from the result of the scanning, two kinds of double-coincidence data can be obtained. For ease of distinguishing between the two kinds of double-coincidence data, the double-coincidence data corresponding to the first tracer is referred to as the first double-coincidence data, and the double-coincidence data corresponding to the second tracer is referred to as the second double-coincidence data.

The first double-coincidence data can be the coincidence event data corresponding to the directly obtained double-coincidence events. The second double-coincidence data can be the coincidence event data corresponding to the double-coincidence events screened from the triple-coincidence events. In other words, when obtaining the first double-coincidence data, the first double-coincidence data can be obtained without screening the triple-coincidence event data. The first double-coincidence data is mainly data in which a pair of *γ* photons, which are converted from an annihilated pair of positive and negative electrons and are almost collinear in opposite directions, is detected simultaneously (or within a coincidence time) by two opposing detectors, the *γ* photons received by the two detectors originating from the decay of the same nuclides; whereas the second double-coincidence data can be obtained only after irrelevant data is removed from the coincidence event data corresponding to the triple-coincidence events.

By performing the PET scan on the subject, pieces of double-coincidence event data respectively corresponding to the double-coincidence events can be obtained.

At Step S1012, the triple-coincidence events are determined from the double-coincidence events based on the corresponding double-coincidence event data, and the first double-coincidence data is determined by screening out, from the double-coincidence event data, the double-coincidence event data corresponding to the triple-coincidence events.

In an embodiment, based on respective sets of double-coincidence event data, the coincidence event data corresponding to the double-coincidence events and the coincidence event data corresponding to the triple-coincidence events can be separated. Specifically, a triple-coincidence event is formed by a combination of three double-coincidence events having repeated detected positions. The format of a single triple-coincidence event can be represented as {(*p*₁, *p*₂, *t*₁₂), (*p*₁, *p*₃, *t*₁₃), (*p*₂, *p*₃, *t*₂₃)}. The ToF of the double-coincidence events from a single triple-coincidence event satisfy the relation *t*₂₃ = *t*₁₃ - *t*₁₂. Based on this, the triple-coincidence event can be screened out from the double-coincidence events. The remaining double-coincidence events are generated by multiple pairs of *γ* photons which are almost collinear in opposite directions, and the double-coincidence event data corresponding to the remaining double-coincidence events can be determined as the first double-coincidence data.

At Step S1013, a prompt gamma photon among the three photons corresponding to each of the triple-coincidence events is determined based on the double-coincidence event data corresponding to the triple-coincidence events, and the second double-coincidence data is obtained based on the double-coincidence event data corresponding to the other two photons other than the prompt gamma photon.

Since multiple pieces of the double-coincidence data corresponding to the triple-coincidence events are mixed with double-coincidence data corresponding to the prompt gamma photon formed upon the emission of a prompt-y ray, in this step, based on a prompt-y event in the triple-coincidence events, the double-coincidence event data, including the prompt gamma photon in the double-coincidence event data corresponding to the triple-coincidence events, is removed. Accordingly, the double-coincidence event data corresponding to the other two photons, other than the prompt gamma photon, can be taken as the second double-coincidence data.

In some embodiments, after the first double-coincidence data and the second double-coincidence data are obtained by screening, the first double-coincidence data and the second double-coincidence data can, after being subjected to data correction (such as dead-time correction), be converted into sinograms and then input into the dual-tracer simultaneous imaging model for subsequent processing.

In this embodiment, by separating the triple-coincidence events from the double-coincidence events and removing the double-coincidence data corresponding to the prompt gamma photon in the data corresponding to the triple-coincidence events, interference data that interferes with nuclear medicine imaging can be effectively reduced, and the accuracy and the iteration efficiency of subsequent iterations of the model can be improved.

In an embodiment, the prompt gamma photon may be identified by performing candidate hypothesis and ToF verification on three photons in a triple-coincidence event. Specifically, among three photon detection positions of three photons in the triple-coincidence event {(*p*₁, *p*₂, *t*₁₂), (*p*₁, *p*₃, *t*₁₃), (*p*₂, *p*₃, *t*₂₃)), one corresponds to the prompt gamma photon, and the other two correspond to the photon pair generated by positron annihilation. Since it is not known in advance which of photon *p*₁, photon *p*₂, and photon *p*₃ is the prompt gamma photon, each of photon *p*₁, photon *p*₂, and photon *p*₃ may be sequentially assumed to be a candidate prompt gamma photon, and the double-coincidence event formed by the remaining two photons may be treated as a candidate true double-coincidence event (i.e., a double-coincidence event originating from positron annihilation), and then the correct hypothesis is determined through ToF verification.

In an embodiment, the ToF verification can include the following Steps S10311 to S10314.

At Step S10311, a candidate decay position is determined based on photon detection positions and a ToF of a candidate true double-coincidence event. Two annihilation photons in a true double-coincidence event originate from the same decay position, propagate in opposite directions, and are detected by two detectors. A ToF of the two photons can reflect a time difference between the two photons arriving at the respective corresponding detectors, and the time difference is associated with a difference between distances from the decay position to the two detectors. Therefore, the candidate decay position can be calculated by weighted summation based on two photon detection positions and the ToF of the candidate true double-coincidence event.

For example, in Step S10311, if photon p3 is assumed to be the candidate prompt gamma photon, the candidate true double-coincidence event is (*p*₁, *p*₂, *t*₁₂), and the candidate decay position p₁₂ may be obtained by *p*₁₂ := *λp*₁ *+* (1 - *λ*)*p*₂. The weight *λ* := (1 - *c* · *t*12/|*p*1 - *p*2|)/2, where c is the speed of light, and |*p*₁ - *p*₂| is the distance between photon *p*₁ and photon *p*₂. Similarly, if *p*₁ is assumed to be the candidate prompt gamma photon, the candidate true double-coincidence event is (*p*₂, *p*₃*, t*₂₃)*,* and the candidate decay position *p*₂₃ := *λ* · *p*₂ + (1 - *λ*) · *p*₃, where the weight *λ* := (1 *- c* · *t*₂₃/|*p*₂ - *p*₃|)/2. If *p*₂ is assumed to be the candidate prompt gamma photon, the candidate true double-coincidence event is (*p*₁, *p*₃, *t*₁₃), and the candidate decay position *p*₁₃ := *λ* · *p*₁ + (1 - *λ*) · *p*₃, where the weight *λ* := (1 - *c* · *t*₁₃/|*p*₁ - *p*₃|)/2.

At Step S10312, calculating estimated ToF data (*t*₁₂', *t*₁₃', *t*₂₃') under the hypothesis based on the candidate decay position, the photon detection positions of the candidate true double-coincidence event, and a photon detection position of the candidate prompt gamma photon. The estimated ToF data represents the theoretical ToF among the three photons under the assumption of the candidate decay position and the candidate prompt gamma photon position.

At Step S10313, comparing the estimated ToF data with measured ToF data. The measured ToF data (*t*₁₂, *t*₁₃, *t*₂₃) may be used as a reference ToF vector, and the estimated ToF data (*t*₁₂', *t*_{13'}, *t*₂₃') may be used as an estimated ToF vector, and a distance between the two vectors (e.g., Euclidean distance) may be calculated as a ToF difference. If the ToF difference is less than a preset difference threshold, it is confirmed that the estimated ToF data under the hypothesis matches the measured ToF data, i.e., the candidate prompt gamma photon under the current hypothesis is the true prompt gamma photon. If the ToF difference is greater than or equal to the difference threshold, it indicates that the current hypothesis is incorrect, and another photon needs to be selected as the candidate prompt gamma photon and the above verification needs to be re-performed.

At Step S10314, after the prompt gamma photon is determined, removing the double-coincidence event data containing the prompt gamma photon from the double-coincidence event data corresponding to the triple-coincidence event, and the remaining one double-coincidence event is the true double-coincidence event originating from positron annihilation. The double-coincidence event data of the true double-coincidence event is the second double-coincidence data.

For those skilled in the art to better understand the above steps, an embodiment of the present application is provided below.

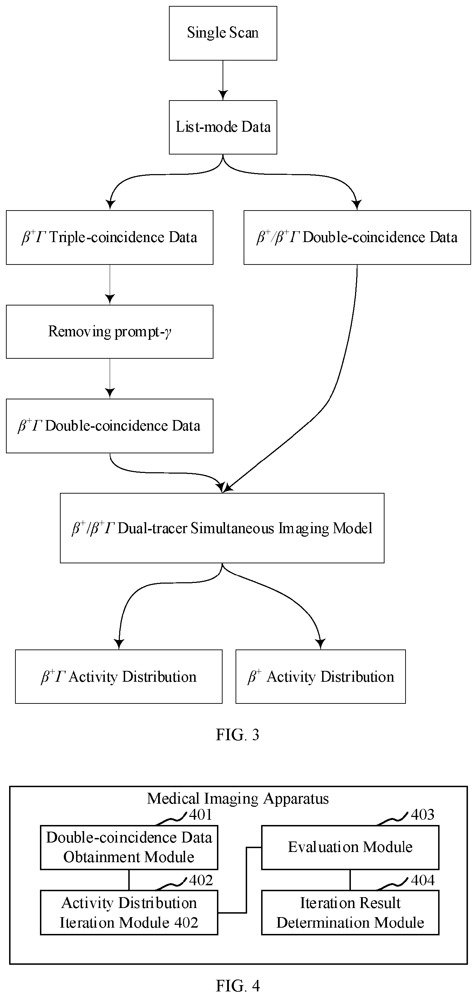

In an embodiment, referring to FIG. 3, in a single scan, pieces of double-coincidence event data (list-mode data) respectively corresponding to multiple double-coincidence events obtained by performing a PET scan on the subject injected with the *β*⁺ tracer (i.e., the first tracer) and the *β*⁺*γ* tracer (i.e., the second tracer) can be obtained. The format of a single piece of list-mode data is (*p*₁, *p*₂, *t*₁₂), where *p*₁ and *p*₂ represent the detected photon positions of two photons, respectively. *t*₁₂ is the ToF between *p*₁ and *p*₂.

Then, the double-coincidence data and the triple-coincidence data can be separated. Specifically, *β*⁺*γ* triple-coincidence events can be extracted from the multiple pieces of list-mode data, and the remaining list-mode data is the *β*⁺/*β*⁺*γ* double-coincidence data (i.e., the first double-coincidence data). A triple-coincidence event is composed of three double-coincidence events having repeated detected photon positions. The format of a single triple-coincidence event is {(*p*₁, *p*₂, *t*₁₂), (*p*₁, *p*₃, *t*₁₃), (*p*₂, *p*₃, *t*₂₃)), and the relationship *t*₂₃ = *t*₁₃ - *t*₁₂ is satisfied.

For the triple-coincidence events, the information of ToF can be configured to determine which of the events *p*₁, *p*₂ and *p*₃ is a photon produced by a prompt-*γ* event, and the double-coincidence events associated with the photon produced by the prompt-*γ* event are removed. The only remaining double-coincidence event is the *β*⁺γ double-coincidence data (i.e., the second double-coincidence data).

Then, the *β*⁺/*β*⁺γ double-coincidence data and the *β*⁺γ double-coincidence data can be corrected and converted into sinograms, denoted as *b*₂ and *b*₃, respectively, where *b*₂ *= b*₂₂ + *b*₃₂, *b*₂₂ includes signals of the *β*⁺ tracer, and *b*₃₂ includes signals of the *β⁺γ* tracer. Then *b*₂ and *b*₃ are input into the dual-tracer simultaneous imaging model. In the dual-subscript naming of *b_{xy} ,* the first subscript *x* indicates which kind of nuclides produce the events contributing to the component (where x=2 corresponds to double-coincidence events produced by the nuclides of the *β*⁺ tracer, and *x* = 3 corresponds to double-coincidence events produced by the nuclides of the *β*⁺*γ* tracer), and the second subscript y indicates to which sinogram group the component belongs (where *y* = 2 corresponds to the sinogram b₂ corresponding to the first double-coincidence data, and *y* = 3 corresponds to the sinogram b₃ corresponding to the second double-coincidence data). In this embodiment, the dual-tracer simultaneous imaging model can be represented by the following Formula (2): ${argmin}_{{f}_{2} , {f}_{3}} \left\{{Fid}_{{b}_{2}}\left({A}_{22}{f}_{2}+{A}_{32}{f}_{3}+{s}_{2}\right)+{Fid}_{{b}_{3}}\left({A}_{3}{f}_{3}+{s}_{3}\right)+{ι}_{+}\left({f}_{2}, {f}_{3}\right)+Reg\left({f}_{2}, {f}_{3}\right)\right\}$ (2) where *f*₂ is the *β*⁺ tracer activity distribution, and *f*₃ is the *β*⁺γ tracer activity distribution; Fid*ᵥ*(*u*) is the fidelity term that measures the deviation between the reconstructed value *u* and the observed value *v*, and is related to a noise distribution. For example, for Poisson noise, Fid*ᵥ*(*u*) = Σ*ᵢ*(*uᵢ* - *vᵢ* ln *uᵢ*) can be taken, and for Gaussian noise, ${Fid}_{v} \left(u\right) = {\left‖u-v\right‖}_{2}^{2}$ can be taken; 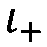 is the positivity constraint; and *s*₂ and *s*₃ are the scatter estimation information obtained by performing scatter estimation on *b*₂ and *b*₃, respectively. *A*₂₂ is a forward projection operator corresponding to the first tracer (the *β*⁺ tracer), *A*₃₂ is a forward projection operator corresponding to the second tracer (the β⁺γ tracer) with respect to the double-coincidence events, and *A*₃ is a forward projection operator corresponding to the second tracer with respect to the double-coincidence events from the triple-coincidence events. The forward projection operators are configured to map a tracer activity distribution from an image space to a measurement space (i.e., a sinogram space), simulating a forward projection process from a known activity distribution to detector measurement data. In an implementation, each forward projection operator may be represented as a system matrix, where elements of the system matrix characterize response contributions of individual voxels in the image space to individual detector pairs in the measurement space (corresponding to a Line of Response, i.e., a Line of Response). The system matrix may be constructed based on system parameters including geometric parameters of detectors of the PET scanning system (such as a dimension of a detector ring, an arrangement manner of detector crystals, and a number of the detector crystals), attenuation correction factors (characterizing an extent to which the *γ* photons are attenuated and absorbed within the subject), and normalization correction factors (characterizing differences in detection efficiency among the detectors). *A*₂₂ maps *f*₂ to expected count values, on Lines of Response, of double-coincidence events produced by decay of the nuclides of the first tracer; *A*₃₂ maps *f*₃ to expected count values, on Lines of Response, of double-coincidence events produced by decay of the nuclides of the second tracer (other than the double-coincidence events extracted from the triple-coincidence events); and *A*₃ maps *f*₃ to expected count values, on Lines of Response, of the double-coincidence events extracted from the triple-coincidence events produced by the nuclides of the second tracer. Since different types of events may correspond to different detection efficiencies and correction factors, *A*₂₂, *A*₃₂, and *A*₃ may be forward projection operators different from one another.

In some embodiments, when iterating *f*₂ and *f*₃, different iteration algorithms can be selected depending on specific circumstances. For example, when Fid*ᵥ* is differentiable, the iteration can be performed by a Fixed-Point Proximity Algorithm, as shown in Formula (3): $\left\{\begin{matrix}{F}^{k + 1} = {x}^{k} {S}^{- 1} \left({ρA}^{T}∇{Fid}_{b}\left({AF}^{k}+s\right)+{Y}^{k}\right) \\ {Y}^{k + 1} = {prox}_{Reg * , T} \left({T}^{- 1}\left(2{F}^{k + 1}-{F}^{k}\right)+{Y}^{k}\right)\end{matrix}\right.$ (3) where *F* : = (*f*₂, *f*₃), *b* : = (*b*₂, *b*₃), *s* : = (*s₂, s*₃), the linear operator *A* is defined as *AF* : = (*A*₂₂*f*₂ + *A*₃₂*f*₃, *A*₃*f*₃ + *s*₃), the positive real number *ρ* is a gradient step size, and *S* and *T* are symmetric positive definite operators. *x*^{k} and *Y*^{k} characterize a primal variable and a dual variable, respectively, in the k-th iteration. x^{k} is a tracer activity distribution obtained by the k-th iteration (that is, a vector F^{k} constituted by the activity distribution of the first tracer f₂ and the activity distribution of the second tracer *f*₃), and *Y*^{k} is a dual variable associated with the regularization term Reg, configured to constrain the iterative process under an action of the proximity operator prox. At the start of the iterations (*k* = 0), *x*⁰ and *Y*⁰ may be initialized to zero or to other reasonable initial values. The gradient step size *ρ* is a positive real number configured to control a magnitude of gradient-based update in each iteration. A value range of *ρ* is related to a Lipschitz constant *L* of a gradient of the fidelity term Fid*ᵥ*; to ensure convergence of the algorithm, the value of *ρ* typically needs to satisfy a condition *ρ* ≤ 1/*L* or another similar convergence condition. In an implementation, *ρ* may take an empirical value (for example, a value between 0.01 and 1), or may be dynamically determined based on a line search or an adaptive stepsize adjustment. The symmetric positive-definite operators *S* and *T* act on a primal variable space and a dual variable space, respectively, and serve as preconditioning operators to improve convergence and numerical stability of the iterations. In an implementation, S and T may respectively be taken as positive scalar multiples of an identity operator I (for example, *S* = σₛ·I and *T* = *σ*ₜ *· I*, where σₛ and σₜ are positive real numbers), in which case the algorithm reduces to a classical primal-dual iteration form. In another implementation, *S* and *T* may be selected based on a structure of the linear operator *A* such that *S - ρA*^{T}*A* remains positive-definite, thereby satisfying convergence conditions of primal-dual algorithms such as Chambolle-Pock. Specifically, the selection of *S* and *T* satisfies a condition *ρ ·* ∥*A*∥² *< σₘᵢₙ*(*S*) *·* σₘᵢₙ(*T*)*,* where ∥*A*∥ is a spectral norm of the linear operator *A,* and *σₘᵢₙ*(*S*) and *σₘᵢₙ(T)* respectively denote a minimum eigenvalue of *S* and a minimum eigenvalue of *T.* Specific values of *S* and *T* may be adjusted according to imaging parameters of the PET scanning system and a type of the selected regularization term. *A*^{T} denotes an adjoint operator of the linear operator *A*; in a case where A is represented as a matrix, *A*^{T} is a transpose matrix of *A. S*⁻¹ and *T*⁻¹ respectively denote an inverse operator of the operator *S* and an inverse operator of the operator *T. ∇Fid_{b}* denotes a gradient operator of the fidelity term *Fid_{b}* with respect to its independent variable, and is a gradient vector of Fid_{b} at a corresponding input point. When *Fid_{b}* corresponds to the Poisson noise case (*Fidᵥ*(*u*) *= Σᵢ*(*uᵢ* - *vᵢlnuᵢ*)), an *i*-th component of *∇Fid_{b}* with respect to *u* is 1 - *v*ᵢ/*u*ᵢ; when *Fid_{b}* corresponds to the Gaussian noise case (*Fidᵥ*(*u*) = ∥*u* - *v*∥²₂), *∇Fid_{b}* = 2(*u - v*). The superscript *k* denotes the *k*-th iteration, where *k* is a non-negative integer. The positivity constraint 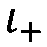 has been defined in Formula (1); in the iterative process shown in Formula (3), 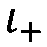 is implicitly applied through an action of the proximity operator prox: *x*^{k} obtained in each iteration is processed by a projection operation that sets negative-valued components of *x*^{k} to zero or maps *x*^{k} into a feasible region in which *f*₂ ≽ 0 and *f*₃ ≽ 0, thereby ensuring that the iterative solution satisfies the physical meaning that the tracer activity distribution is non-negative.

The conjugate Reg* of the function Reg is defined by Formula (4): $Reg * \left(u\right) : = {sup}_{x} \left\{\left〈x, u\right〉-Reg\left(x\right)\right\}$ (4) where ${sup}_{x}$ denotes taking a supremum over the variable *x,* and 〈*x, u*〉 denotes an inner product between a vector *x* and a vector *u.*

The proximity operator prox*_{g,T}* of a function *g* is defined by Formula (5): ${prox}_{g , T} \left(u\right) : = {argmin}_{u} \left\{g\left(u\right)+\frac{1}{2}{\left‖u-x\right‖}_{T}^{2}\right\}$ (5) where argmin*ᵤf*(*u*) is the minimum point of the function *f, T* is a positive-definite self-adjoint operator, and ${\left‖u\right‖}_{T} : = \sqrt{\left〈u, Tu\right〉}$ is the *T*-weighted norm. g is a proper convex function defined on a corresponding variable space (for example, the regularization term *Reg* or its conjugate function Reg* mentioned above); *argmin*ᵤ*f*(*u*) denotes a minimum point of a function *f* ; T is a self-adjoint positive-definite operator, that is, *T* satisfies *T = T*^{T} and a quadratic form corresponding to *T* is positive; and ∥ · ∥*_{T}* is a T-weighted norm.

Although the steps in the flowcharts involved in the above embodiments are sequentially shown as indicated by the arrows, these steps are not necessarily performed sequentially in the order indicated by the arrows. Unless explicitly specified herein, there is no strict order limitation on the execution of these steps, and these steps may be performed in other orders. Moreover, at least part of the steps in the flowcharts involved in the above embodiments may include a plurality of steps or a plurality of stages, which are not necessarily performed at the same moment, but may be performed at different moments. The execution order of these steps or stages is not necessarily sequential, but may be performed alternately or in turn with at least part of other steps or of steps or stages within other steps.

Based on the same inventive concept, the embodiments of the present application further provide a medical imaging apparatus for implementing the above-mentioned medical imaging method. The solution to the problem solved by the apparatus is similar to the solution described in the above-mentioned method, and therefore, the specific definitions in one or more embodiments of the medical imaging apparatus provided below can be found in the above definitions on the medical imaging method, and will not be repeated.

In an embodiment, as shown in FIG. 4, a medical imaging apparatus is provided, including a double-coincidence data obtainment module 401, an activity distribution iteration module 402, an evaluation module 403, and an iteration result determination module 404.

The double-coincidence data obtainment module 401 is configured to obtain the first double-coincidence data of the subject and the second double-coincidence data of the subject. The first double-coincidence data is determined based on double-coincidence events generated by the nuclides in the first tracer and double-coincidence events generated by the nuclides in the second tracer in the subject. The second double-coincidence data is determined based on double-coincidence events from triple-coincidence events generated by the nuclides in the second tracer.

The activity distribution iteration module 402 is configured to obtain, by iterations, activity distributions of the first tracer and activity distributions of the second tracer, based on the first double-coincidence data and the second double-coincidence data.

The evaluation module 403 is configured to obtain, for each of the iterations, the respective evaluation parameter reflecting the accuracy of the iteration, based on the activity distribution of the first tracer and the activity distribution of the second tracer both obtained in the iteration.

The iteration result determination module 404 is configured to determine the target activity distribution of the first tracer and the target activity distribution of the second tracer, based on the evaluation parameters respectively corresponding to the iterations, when the stopping criterion for iteration is satisfied.

In an embodiment, the evaluation module 403 is configured to determine the reconstruction result of the first double-coincidence data based on the activity distribution of the first tracer and the activity distribution of the second tracer obtained by the iteration, and determine the reconstruction result of the second double-coincidence data based on the activity distribution of the second tracer obtained by the iteration; determine the fidelity term of the current iteration based on the first difference between the reconstruction result of the first double-coincidence data and the first double-coincidence data, and the second difference between the reconstruction result of the second double-coincidence data and the second double-coincidence data; determine a corresponding regularization term and a positivity constraint term for constraining a value range of the activity distribution of the first tracer and the activity distribution of the second tracer, based on the activity distribution of the first tracer and the activity distribution of the second tracer obtained by the iteration; and determine the evaluation parameter reflecting the accuracy of each iteration based on a sum of the fidelity term, the regularization term and the positivity constraint term corresponding to the iteration.

In an embodiment, the evaluation module 403 is configured to determine the reconstruction result of the first double-coincidence data based on the activity distribution of the first tracer obtained by the iteration, the first preset influence factor of the activity distribution of the first tracer on the first double-coincidence data, the activity distribution of the second tracer obtained by the iteration, and the second preset influence factor of the activity distribution of the second tracer on the first double-coincidence data; and determine the reconstruction result of the second double-coincidence data based on the activity distribution of the second tracer obtained by the iteration and the third preset influence factor of the activity distribution of the second tracer on the second double-coincidence data.

In an embodiment, the evaluation module 403 is configured to determine the double-coincidence data reconstruction result of the double-coincidence events generated by the nuclides in the first tracer based on a product of the activity distribution of the first tracer obtained by the iteration and the first preset influence factor of the activity distribution of the first tracer on the first double-coincidence data; determine the double-coincidence data reconstruction result of the double-coincidence events generated by the nuclides in the second tracer based on the product of the activity distribution of the second tracer obtained by the iteration and the second preset influence factor of the activity distribution of the second tracer on the first double-coincidence data; and determine the reconstruction result of the first double-coincidence data based on the double-coincidence data reconstruction result of the double-coincidence events generated by the nuclides in the first tracer and the double-coincidence data reconstruction result of the double-coincidence events generated by the nuclides in the second tracer.

In an embodiment, the evaluation module 403 is configured to obtain the first scatter estimation information corresponding to the first double-coincidence data; and obtain the reconstruction result of the first double-coincidence data based on the sum of the double-coincidence data reconstruction result of the double-coincidence events generated by the nuclides in the first tracer, the double-coincidence data reconstruction result of the double-coincidence events generated by the nuclides in the second tracer, and the first scatter estimation information.

In an embodiment, the evaluation module 403 is configured to determine the double-coincidence data reconstruction result corresponding to the double-coincidence events from the triple-coincidence events of the second tracer based on a product of the activity distribution of the second tracer obtained by the iteration and the third preset influence factor of the activity distribution of the second tracer on the second double-coincidence data; and determine the reconstruction result of the second double-coincidence data based on the double-coincidence data reconstruction result corresponding to the double-coincidence events from the triple-coincidence events of the second tracer.

In an embodiment, the evaluation module 403 is configured to obtain the second scatter estimation information corresponding to the second double-coincidence data; and obtain the reconstruction result of the second double-coincidence data based on the sum of the double-coincidence data reconstruction result corresponding to the double-coincidence events from the triple-coincidence events of the second tracer and the second scatter estimation information.

In an embodiment, the double-coincidence data obtainment module 401 is configured to obtain the double-coincidence event data respectively corresponding to the double-coincidence events of the subject injected with the first tracer and the second tracer. The nuclides of the first tracer produce the double-coincidence events. The nuclides of the second tracer produce the double-coincidence events and the triple-coincidence events. Each piece of the double-coincidence event data includes the two detected photon positions and the ToF between the two detected photon positions; based on the respective pieces of double-coincidence event data, determine triple-coincidence events from the double-coincidence events, and determine the first double-coincidence data by screening out, from the double-coincidence event data, the double-coincidence event data corresponding to the triple-coincidence events; and based on the double-coincidence event data corresponding to the triple-coincidence events, determine the prompt gamma photon among the three photons corresponding to each of the triple-coincidence events, and obtain the second double-coincidence data based on the double-coincidence event data corresponding to the other two photons other than the prompt gamma photon.

The various modules in the above medical imaging apparatus can be implemented entirely or partially by software, hardware, or a combination thereof. The various modules can be embedded in, or independent of, a processor in a computer device in the form of hardware, or can be stored in a memory of the computer device in the form of software, so that the processor can invoke and execute the operations corresponding to the various modules.

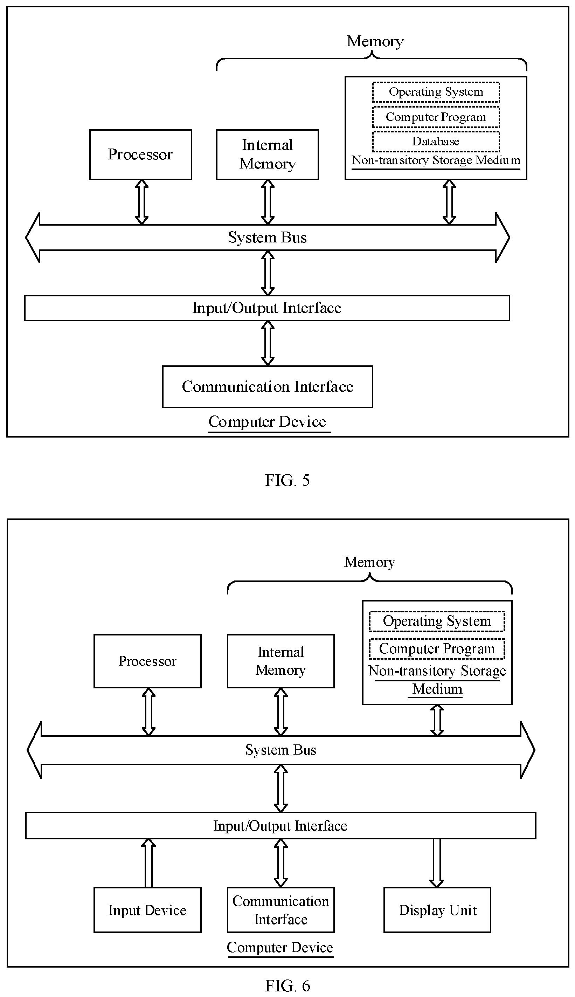

In an embodiment, a computer device is provided. The computer device may be a server. An internal structure diagram thereof can be as shown in FIG. 5. The computer device includes a processor, a memory, an input/output (I/O) interface, and a communication interface. The processor, the memory, and the input/output interface are connected through a system bus, and the communication interface is connected to the system bus through the input/output interface. The processor of the computer device is configured to provide computing and control capabilities. The memory of the computer device includes a non-transitory storage medium and an internal memory. The non-transitory storage medium stores an operating system, a computer program, and a database. The internal memory provides an environment for the operation of the operating system and the computer program in the non-transitory storage medium. The database of the computer device is configured to store nuclear medicine imaging data. The input/output interface of the computer device is configured to exchange information between the processor and an external device. The communication interface of the computer device is configured to communicate with an external terminal through a network connection. The computer program, when executed by the processor, causes the processor to perform a medical imaging method.

In an embodiment, a computer device is provided. The computer device may be a terminal, and an internal structure diagram thereof can be as shown in FIG. 6. The computer device includes a processor, a memory, an input/output interface, a communication interface, a display unit, and an input device. The processor, the memory, and the input/output interface are connected through a system bus, and the communication interface, the display unit, and the input device are connected to the system bus through the input/output interface. The processor of the computer device is configured to provide computing and control capabilities. The memory of the computer device includes a non-transitory storage medium and an internal memory. The non-transitory storage medium stores an operating system and a computer program. The internal memory provides an environment for the operation of the operating system and the computer program in the non-transitory storage medium. The input/output interface of the computer device is configured to exchange information between the processor and an external device. The communication interface of the computer device is configured to communicate with an external terminal in a wired or wireless manner, and the wireless manner may be implemented through Wi-Fi, a mobile cellular network, Near Field Communication (NFC), or other techniques. The computer program, when executed by the processor, causes the processor to perform the medical imaging method. The display unit of the computer device is configured to form visually perceptible images, and may be a display screen, a projection device, or a virtual reality imaging device. The display screen may be a liquid crystal display or an electronic ink display. The input device of the computer device may be a touch layer covering the display screen, a key, a trackball, or a touchpad disposed on the housing of the computer device, or an external keyboard, touchpad, or mouse.

Those skilled in the art can understand that the structures shown in FIG. 5 and FIG. 6 are only block diagrams of partial structures related to the solution of the present application, and do not constitute a limitation on the computer device to which the solution of the present application is applied. The specific computer device may include more or fewer components than those shown in the drawings, combine some components, or have different component arrangements.

In an embodiment, a computer device is provided, including a memory and a processor. The memory stores a computer program. The processor, when executing the computer program, performs the steps of the medical imaging method provided in the above embodiments.

In an embodiment, a non-transitory computer-readable storage medium is provided, having a computer program stored thereon. The computer program, when executed by a processor, causes the processor to perform the steps of the medical imaging method provided in the above embodiments.

In an embodiment, a computer program product is provided, including a computer program. The computer program, when executed by a processor, causes the processor to perform the steps of the medical imaging method provided in the above embodiments.

It should be noted that user information (including but not limited to user device information, user personal information, etc.) and data (including but not limited to data for analysis, stored data, displayed data, etc.) involved in the present application are all information authorized by users or fully authorized by all parties, and the collection, use and processing of the relevant data need to comply with the relevant laws and regulations.

Those of ordinary skill in the art can understand that all or part of the processes of implementing the medical imaging method in the above embodiments can be implemented by instructing relevant hardware through a computer program. The computer program can be stored in a non-transitory computer-readable storage medium. When executed, the computer program may include the processes of the medical imaging method provided in the above embodiments. Any reference to the memory, database, or other medium used in the embodiments provided in the present application can include at least one of a non-transitory memory and a transitory memory. The non-transitory memory may include Read-Only Memory (ROM), magnetic tape, floppy disk, flash memory, optical memory, high-density embedded non-transitory memory, Resistive Random Access Memory (ReRAM), Magnetoresistive Random Access Memory (MRAM), Ferroelectric Random Access Memory (FRAM), Phase Change Memory (PCM), graphene memory, and the like. The transitory memory may include Random Access Memory (RAM) or external cache memory, or the like. By way of explanation and not limitation, RAM may be in various forms, such as Static Random Access Memory (SRAM) or Dynamic Random Access Memory (DRAM). The database involved in the embodiments of the present application can include at least one of a relational database and a non-relational database. The non-relational database may include a blockchainbased distributed database, without being limited thereto. The processor involved in the embodiments of the present application may be a general-purpose processor, a central processing unit, a graphics processing unit, a digital signal processor, a programmable logic device, a data processing logic device based on quantum computing, an artificial intelligence (AI) processor, or the like, without being limited thereto.

The technical features of the above embodiments can be combined arbitrarily. For the sake of brevity, not all possible combinations of the technical features in the above embodiments have been described. However, as long as there is no contradiction between the combinations of these technical features, they should all be considered to fall within the scope described in the present application.

The above embodiments merely express several implementations of the technical solution provided in the present application. The description is detailed, but it should not be construed as limiting the scope of the present application. It should be noted that, for those of ordinary skill in the art, some modifications and improvements can be made without departing from the inventive concept of the technical solution provided in the present application.

## Claims

1. A medical imaging method, **characterized by**, comprising:
obtaining first double-coincidence data and second double-coincidence data of a scanned subject, wherein the first double-coincidence data is determined based on double-coincidence events generated by nuclides in a first tracer and double-coincidence events generated by nuclides in a second tracer in the subject, and the second double-coincidence data is determined based on double-coincidence events from triple-coincidence events generated by the nuclides in the second tracer;
obtaining, by iterations, a target activity distribution of the first tracer and a target activity distribution of the second tracer, based on the first double-coincidence data and the second double-coincidence data, wherein for each of the iterations, a respective evaluation parameter is obtained based on an activity distribution of the first tracer and an activity distribution of the second tracer both obtained in each of the iterations, and the target activity distribution of the first tracer and the target activity distribution of the second tracer are determined based on evaluation parameters corresponding to the iterations, respectively, when a stopping criterion for the iterations is satisfied.

2. The medical imaging method according to claim 1, wherein obtaining, for each of the iterations, the respective evaluation parameter based on the activity distribution of the first tracer and the activity distribution of the second tracer both obtained in each of the iterations comprises:
determining a fidelity term of each of the iterations, based on the activity distribution of the first tracer and the activity distribution of the second tracer both obtained by each of the iterations;
determining a regularization term and a positivity constraint term for constraining a value range of the activity distribution of the first tracer and a value range of the activity distribution of the second tracer, based on the activity distribution of the first tracer and the activity distribution of the second tracer both obtained by each of the iterations; and
determining the evaluation parameter based on a sum of the fidelity term, the regularization term, and the positivity constraint term all corresponding to each of the iterations

3. The medical imaging method according to claim 2, wherein determining the fidelity term of each of the iterations, based on the activity distribution of the first tracer and the activity distribution of the second tracer both obtained by each of the iterations comprises:
determining a reconstruction result of the first double-coincidence data based on the activity distribution of the first tracer and the activity distribution of the second tracer both obtained by each of the iterations, and determining a reconstruction result of the second double-coincidence data based on the activity distribution of the second tracer obtained by each of the iterations; and
determining the fidelity term of each of the iterations, based on a first difference between the reconstruction result of the first double-coincidence data and the first double-coincidence data and a second difference between the reconstruction result of the second double-coincidence data and the second double-coincidence data

4. The medical imaging method according to claim 3, wherein determining the reconstruction result of the first double-coincidence data based on the activity distribution of the first tracer and the activity distribution of the second tracer both obtained by each of the iterations, and determining the reconstruction result of the second double-coincidence data based on the activity distribution of the second tracer obtained by each of the iterations, comprises:
determining the reconstruction result of the first double-coincidence data based on the activity distribution of the first tracer obtained by each of the iterations, a first preset influence factor of the activity distribution of the first tracer on the first double-coincidence data, the activity distribution of the second tracer obtained by each of the iterations, and a second preset influence factor of the activity distribution of the second tracer on the first double-coincidence data; and
determining the reconstruction result of the second double-coincidence data based on the activity distribution of the second tracer obtained by each of the iterations and a third preset influence factor of the activity distribution of the second tracer on the second double-coincidence data.

5. The medical imaging method according to claim 4, wherein determining the reconstruction result of the first double-coincidence data based on the activity distribution of the first tracer obtained by each of the iterations, the first preset influence factor of the activity distribution of the first tracer on the first double-coincidence data, the activity distribution of the second tracer obtained by each of the iterations, and the second preset influence factor of the activity distribution of the second tracer on the first double-coincidence data comprises:
determining a reconstruction result of double-coincidence data of the double-coincidence events generated by the nuclides in the first tracer, based on a product of the activity distribution of the first tracer obtained by each of the iterations and the first preset influence factor of the activity distribution of the first tracer on the first double-coincidence data;
determining a reconstruction result of double-coincidence data of the double-coincidence events generated by the nuclides in the second tracer, based on a product of the activity distribution of the second tracer obtained by each of the iterations and the second preset influence factor of the activity distribution of the second tracer on the first double-coincidence data; and
determining the reconstruction result of the first double-coincidence data based on the reconstruction result of double-coincidence data of the double-coincidence events generated by the nuclides in the first tracer and the reconstruction result of double-coincidence data of the double-coincidence events generated by the nuclides in the second tracer.

6. The medical imaging method according to claim 5, wherein determining the reconstruction result of the first double-coincidence data based on the reconstruction result of double-coincidence data of the double-coincidence events generated by the nuclides in the first tracer and the reconstruction result of double-coincidence data of the double-coincidence events generated by the nuclides in the second tracer comprises:
obtaining first scatter estimation information corresponding to the first double-coincidence data; and
obtaining the reconstruction result of the first double-coincidence data based on a sum of the reconstruction result of double-coincidence data of the double-coincidence events generated by the nuclides in the first tracer, the reconstruction result of double-coincidence data of the double-coincidence events generated by the nuclides in the second tracer, and the first scatter estimation information.

7. The medical imaging method according to claim 4, wherein determining the reconstruction result of the second double-coincidence data based on the activity distribution of the second tracer obtained by each of the iterations and the third preset influence factor of the activity distribution of the second tracer on the second double-coincidence data comprises:
determining the reconstruction result of double-coincidence data corresponding to the double-coincidence events from the triple-coincidence events of the second tracer, based on a product of the activity distribution of the second tracer obtained by each of the iterations and the third preset influence factor of the activity distribution of the second tracer on the second double-coincidence data; and
determining the reconstruction result of the second double-coincidence data based on the reconstruction result of double-coincidence data corresponding to the double-coincidence events from the triple-coincidence events of the second tracer.

8. The medical imaging method according to claim 7, wherein determining the reconstruction result of the second double-coincidence data based on the reconstruction result of double-coincidence data corresponding to the double-coincidence events from the triple-coincidence events of the second tracer comprises:
obtaining second scatter estimation information corresponding to the second double-coincidence data; and
obtaining the reconstruction result of the second double-coincidence data based on a sum of the reconstruction result of double-coincidence data corresponding to the double-coincidence events from the triple-coincidence events of the second tracer and the second scatter estimation information.

9. The medical imaging method according to claim 1, wherein obtaining the first double-coincidence data and the second double-coincidence data of the scanned subject comprises:
obtaining double-coincidence event data corresponding to the double-coincidence events of the subject injected with the first tracer and the second tracer, wherein the nuclides of the first tracer produce double-coincidence events, the nuclides of the second tracer produce double-coincidence events and triple-coincidence events, and each piece of the double-coincidence event data comprises two detected photon positions and a time of flight between the two detected photon positions;
determining the triple-coincidence events from the double-coincidence events based on the double-coincidence event data, and determining the first double-coincidence data by screening out, from the double-coincidence event data, the double-coincidence event data corresponding to the triple-coincidence events; and
determining, based on the double-coincidence event data corresponding to the triple-coincidence events, a prompt gamma photon among three photons corresponding to each of the triple-coincidence events, and obtaining the second double-coincidence data based on the double-coincidence event data corresponding to the other two photons other than the prompt gamma photon.

10. The medical imaging method according to any one of claims 1-9, wherein the first tracer and the second tracer are injected simultaneously or injected with an injection interval less than a preset threshold, and the preset threshold is determined based on the accuracy of the target activity distribution of the first tracer and the accuracy of the target activity distribution of the second tracer obtained under different injection intervals.

11. The medical imaging method according to any one of claims 1-10, wherein an algorithm configured for the iterations is one of a fixed-point proximity algorithm, a splitting algorithm, a primal-dual algorithm, an inexact Uzawa algorithm, an alternating direction algorithm of multipliers, or an ordered subsets expectation maximization algorithm.

12. The medical imaging method according to any one of claims 4-11, wherein the first preset influence factor is a forward projection operator that maps the activity distribution of the first tracer to the double-coincidence event data generated by the nuclides of the first tracer.

13. The medical imaging method according to any one of claim 4-12, wherein the second preset influence factor is a forward projection operator that maps the activity distribution of the second tracer to the double-coincidence event data generated by the nuclides of the second tracer.

14. A non-transitory storage medium having a computer program stored thereon, wherein the computer program, when executed by a processor, causes the processor to perform the medical imaging method according to any one of claims 1-13.

15. A computer device comprising a memory and a processor, wherein the memory stores a computer program, and the processor, when executing the computer program, performs the medical imaging method according to any one of claims 1-13.
